Europäisches Patentamt

(19) European Patent Office

Office européen des brevets

(11) Publication number: **0 042 405**
**B1**

(12) **EUROPEAN PATENT SPECIFICATION**

(45) Date of publication of patent specification: **25.07.84**

(51) Int. Cl.³: **A 61 B 5/02**

(21) Application number: **81900198.3**

(22) Date of filing: **23.12.80**

(86) International application number:
**PCT/HU80/00011**

(87) International publication number:
**WO 81/01790 09.07.81 Gazette 81/16**

(54) **APPARATUS FOR MEASURING BLOOD PRESSURE OF LABORATORY ANIMALS.**

| | |
|---|---|
| (30) Priority: **27.12.79 HU VI001285** | (73) Proprietor: **VILLAMOSIPARI KUTATO INTEZET**<br>**Cservenka Miklós ut 86**<br>**H-1601 Budapest XV (HU)** |
| (43) Date of publication of application:<br>**30.12.81 Bulletin 81/52** | (73) Proprietor: **GYOGYSZERKUTATO INTEZET**<br>**Szabadsàgharcosok utja 47-49**<br>**Budapest 1045 (HU)** |
| (45) Publication of the grant of the patent:<br>**25.07.84 Bulletin 84/30** | (72) Inventor: **DANITZ, Béla**<br>**Pozsonyi u. 2/a**<br>**H-1215 Budapest (HU)**<br>Inventor: **GLOFAK, F. Péter**<br>**Szörény u. 5-7**<br>**H-1087 Budapest (HU)**<br>Inventor: **KISS, Miklós**<br>**Bocskay u. 4-6**<br>**H-1114 Budapest (HU)** |
| (84) Designated Contracting States:<br>**AT CH DE FR GB LI LU NL SE** | |
| (56) References cited:<br>**DE - A - 2 555 453**<br>**FR - A - 2 342 649**<br>**US - A - 4 030 485**<br>**US - A - 4 050 452**<br><br>**Journal of Applied Phisiology, volume 25, N 6, published May 1968, Dowd D.A. et al "A method for recording baby rat rystolic blood pressures", see pages 772-774** | (74) Representative: **Gold, Tibor Zoltán et al,**<br>**T.Z.GOLD & COMPANY 9, Staple Inn**<br>**London WC1V 7QH (GB)** |

Courier Press, Leamington Spa, England.

(56) References cited:
Catalogue of the Ministry of Health of the USSR
"Meditsinskie instrumenty, pribory, apparaty i
oborudovanie" book 1, published 1961,
Vneshtorgizdatelstvo, see page 428, fig 03-28
B.A. Kuznetsov, "Dicherazvedenie" published
1972, Publishing House Lesnaya
promyshlennost, see page 108
G.I.T. FACHZEITSCHRIFT FÜR DAS
LABORATORIUM, vol. 18, no. 8, August 1974,
Darmstadt, DE, Fischer-Labortechnik
"Elektronisches Blutdruck- und
Pulsfrequenzmessgerät für Versuchstiere",
pages 790-791

## Description

The invention relates to the field of laboratory pharmacology, more particularly to an apparatus for measuring blood pressure of laboratory animals, principally to detect the effects of antihypertensive drugs.

An important step in researching anti-hypertensive drugs is to study the effects of new materials in tests with laboratory animals. These tests have statistical character and they have to be carried out on a large number of animals over a long period of time. For such tests a blood pressure measuring equipment is required, by which the blood pressure of the laboratory animals can be taken at any moment without any damage to the animal during the treatment, which can last for several weeks or months. To carry out the measurements fast and precisely, taking as little human work as possible and to provide results evaluated statistically form the subject of further requirements.

The so-called Heatless method (referred to e.g. in the catalogue of the Innovators Instrumentation Co. U.S. relating to the "Indirect Blood Pressure Test System for Rats") is known as a non-invasive technique for measuring blood pressure. According to this method, a sensor cuff containing an optical signal source and a sensor is fastened on the tail of a rat located in stocks. As the heat exchange of the rats is low at the employed temperature, the pulsation of the tail-veins is weak. The measured data are recorded in appropriate form by which skilled persons can draw conclusions for the systolic blood pressure.

In such individual measurements each rat has to be held down separately to ensure that its tail is still during the measurement. The trembling of the tail and other factors, however, make the measurement uncertain and a lot of skill and patience are required to obtain acceptable results.

Owing to the great number of measurements which are to be carried out, automatic measurements have been considered desirable, but automatic measurements have been considered so far to be unaccomplishable because of the adverse noise, movement and other disturbing effects associated therewith. Automatic measurements have been actually carried out in cases where the laboratory animal had been given sedatives or been anaesthetized previously to eliminate movements interfering with the measurement. The object of most of the tests, however, is to detect the influence of certain drugs on blood pressure, and this influence is dependent also on the sedative or narcotic applied.

From the Journal of Applied Physiology, Vol. 25, No. 6, December 1968, pages 772—774, a device for measuring blood pressure of a laboratory animal is known, comprising a combined clamping and sensor unit fastened on the tail of the laboratory animal, a measuring channel for processing output signals of the sensor, the device further comprising a pressure developer and distributor equipment and air conduits, coupling the combined clamping and sensor units to said equipment. From US—A—4,050,452 it is known to control the pressure in a cuff of a device for measuring blood pressure via a differential pressure switch in order to develop predetermined periodical pressure changes in said cuff. From US—A—4,030,485 a device for measuring blood pressure is known, comprising an optical sensor unit.

## Object of the invention

The object of the present invention as claimed is to provide a device for measuring blood pressure on a plurality of laboratory animals automatically and simultaneously, that can eliminate the need for using sedatives.

## Disclosure of the invention

The basic principle of the present invention lies in the way of suppressing and eliminating the effects interfering with the measurement. The signal-to-noise ratio of the pulse-detection can be increased considerably by raising the temperature of the laboratory animals, e.g. by heating the bottom of their cages (to a temperature range of 33 to 35°C), because it intensifies their circulation. Such a heating causes about a 10% consistent increase in blood pressure.

According to another aspect of the present invention the pressure is changed simultaneously for all of the laboratory animals by means of a common pressure developer and distributor equipment. The signals of the individual pulse sensors are processed in separate measuring channels and the measuring channels comprise a matching amplifier, a low-pass filter with a cut-off level of about 80 dB/decade and a cut-off frequency of not more than 10 Hz, AC-coupled amplifiers, a comparator and a decoupled coupling unit.

The filtered signals of the measuring channel are processed in several measuring phases as follows. When the first measuring signal is sensed, the corresponding pressure is recorded and it is considered as the blood pressure if at least a given number (e.g. 4) of signals are sensed during each one of a number of subsequent periods with predetermined length (e.g. during periods of 1 second). The number of the periods can be e.g. six.

A measurement series consisting of several measurement periods is accepted only if a given percentage (e.g. 70—80 per cent) of its periods supplies valuable signals. A signal is valuable if it lies within a given (e.g. a 10 per cent) variance range of an average value derived from the measurement series. If this condition is not met the measurement series will be repeated a given number of times, e.g. not more than three times.

Brief description of drawings

Figure 1 shows the schematic block diagram of the apparatus of the present invention;

Figure 2 shows an enlarged sectional elevation view of the combined clamping and sensor unit of the apparatus of Figure 1;

Figure 3 is a sectional view taken along line III—III of Figure 2;

Figure 4 shows the block diagram of the stages associated with a measuring channel; and

Figure 5 is a time diagram of pressure and pulse signals during one measuring series.

Best mode of carrying out the invention

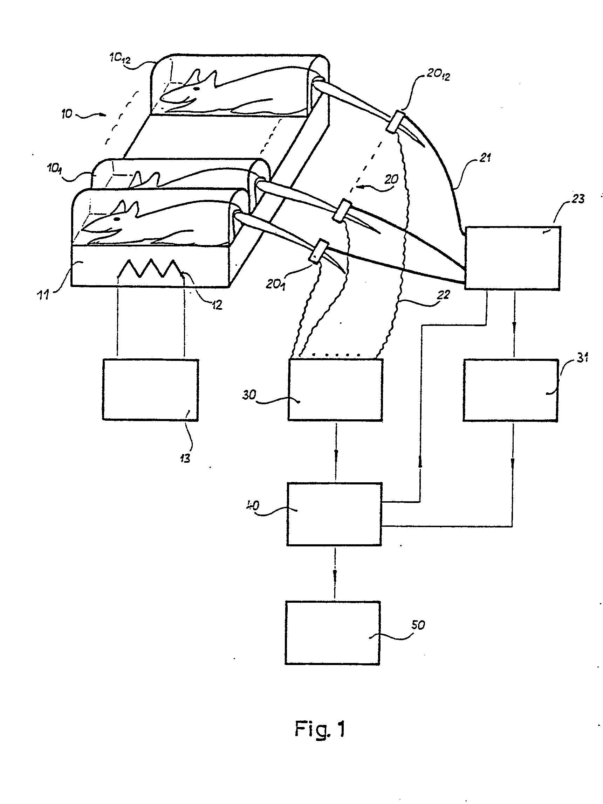

Referring to Figure 1, there is shown the block diagram of an apparatus for measuring blood pressure of rats. This apparatus comprises twelve measuring locations with respective measuring cages $10_1...10_{12}$ arranged like stocks. The measuring cages restrict the free movement of the laboratory animals and the tails of the laboratory animals extend out from the cages.

The measuring cages 10 have a common bottom 11 heated to a temperature of about 34°C for the duration of the measurements by means of a heater 12 and a temperature regulator unit 13. As a result of the heating the blood circulation of the laboratory animal will be increased, facilitating the measurement.

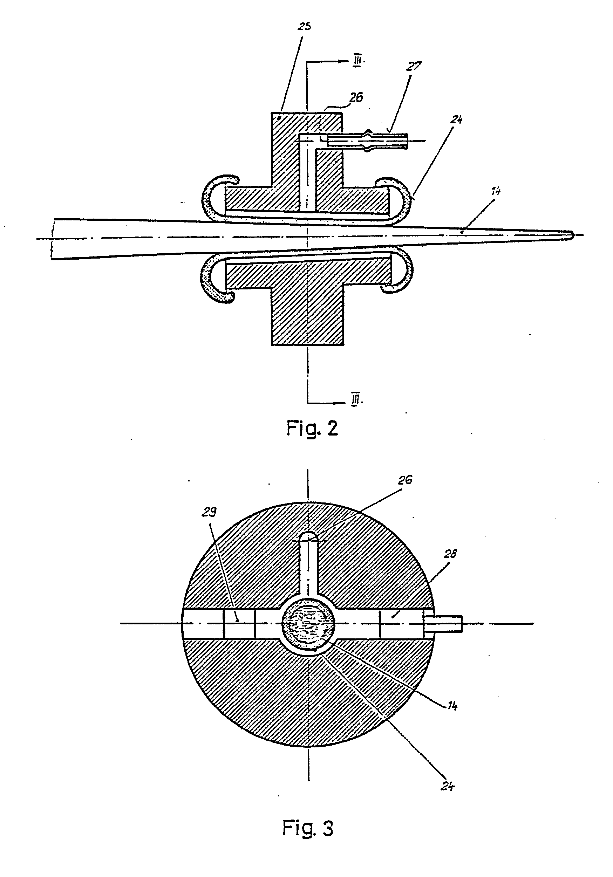

Respective combined clamping and optical sensor units $20_1...20_{12}$ are fastened on the tail of each laboratory animal. A preferable embodiment of such a unit is shown in Figures 2 and 3. Respective air conduits 21 and signal lines 22 are connected to each combined clamping and optical sensor unit 20. The air conduits 21 are coupled to a common pressure developer and distributor equipment 23 that receives electric control signals from a program controller and evaluating unit 40. The signal lines 22 are coupled to inputs of a measuring and filter unit 30. The pressure developer and distributor equipment 23 comprises a compressor that increases the pressure in the interior of a pressure distributor of the equipment of a given rate in response to a control signal. There is provided a narrow opening formed by a needle valve in the pressure distributing space of the equipment 23 that ensures a pressure drop at a given rate. A pressure transducer 31 is coupled to the distributor space, and it supplies an electric output signal proportional to the pressure prevailing in the space, and this signal is connected to one of the inputs of the program controller and evaluating unit 40.

The measuring and filter unit 30 is controlled by the program controller and evaluating unit 40 preferably comprising a microprocessor. The measured data are recorded by data recording equipment 50, e.g. by a line printer connected to the program controller and evaluating unit 40.

(I) Reference is made now to Figures 2 and 3, wherein the arrangement of the combined clamping and sensor unit 20 is shown. The tail 14 of the laboratory animal is girdled on a given length by a cuff 24 made of elastic material, e.g. of rubber. Both ends of the cuff 24 are connected tightly to two ends of a rigid carrier ring 25. An air channel 26 is formed inside the carrier ring 25 that is connected to a channel formed within the carrier ring on one side of the cuff 24. An air duct 27 communicates with the air channel 26 and is adapted for receiving the end of the air conduit 21 (see Figure 1).

In the middle of the carrier ring 25, a transverse bore is provided in which a light source 28 and a light sensor 29 are arranged in opposite positions.

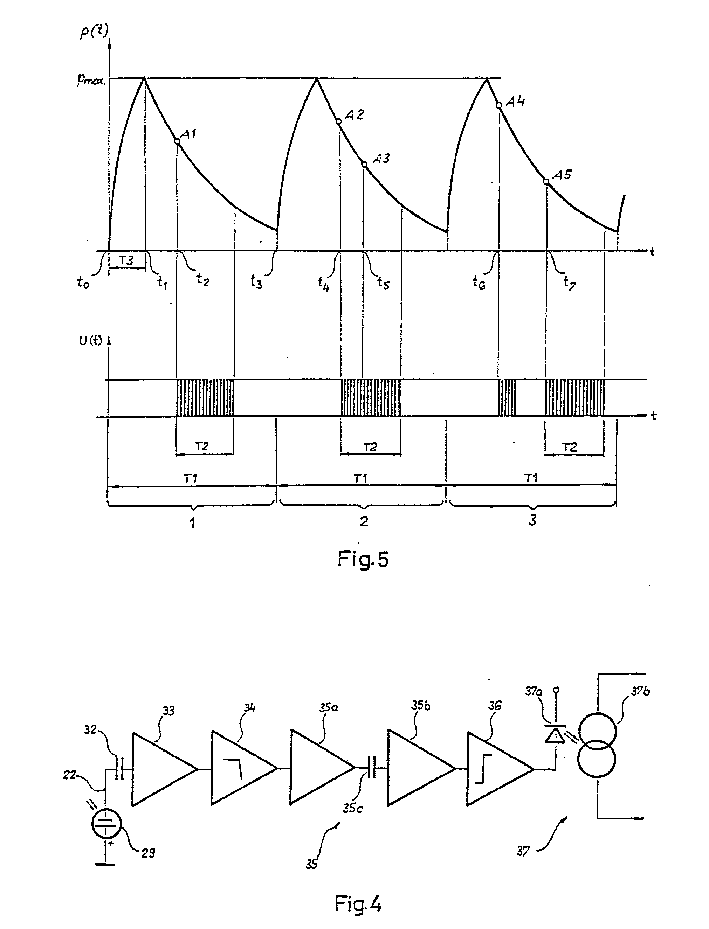

Reference is made now to Figure 4 in which the block diagram of a measuring channel of the measuring and filter unit 30 is shown. The light sensor 29 coupled to the input of the measuring and filter unit 30 through the signal line 22 has also been shown in Figure 4. The measuring channel comprises a capacitor 32 for DC decoupling, a matching amplifier 33 of low-pass characteristics connected with its input to the capacitor 32, a low-pass filter 34, a pair of amplifiers 35a and 35b connected to the output of the filter 34, a capacitor 35c for DC separation between the amplifiers, a comparator 36 and a coupling unit 37 with DC separation, comprising preferably a light emitting diode 37a and a current generator 37b controlled by the light of the diode 37a.

The operation of the blood pressure measuring system of the present invention will be described with reference to the time diagrams of Figure 5.

Before the beginning of the measurements the laboratory animals are located in the respective measuring cages 10, and the combined clamping and optical sensor units 20 are fastened on their tails. The heating is switched on, and some time thereafter the temperature of the animals reaches an elevated constant value. Then the compressor of the pressure developer and distributor equipment 23 is switched on in the moment $t_0$, and from this moment the pressure in the pressure distributor, as well as in the air channels 26 of each sensor 20 increases exponentially. The pressure transducer 31 converts the increase in pressure to an electric signal, whose curve section defines the period T3 in Figure 5. The initial rate of increase in pressure is about 3—4000 Pa/sec and the pressure keeps on increasing until it reaches a predetermined maximum value $P_{max}$. The maximum pressure $P_{max}$ is set by the program controller and evaluating unit 40 and it can vary between about $1,066 \times 10^5$ Pa and $1,4 \times 10^5$ Pa, depending on the actual laboratory circumstances. The typical value is $1,33 \times 10^5$ Pa. The measurement starts at the

moment $t_1$, when the compressor is switched off and the pressure in the system decreases exponentially at a rate of about 3000 Pa/sec as the air flows out through the needle valve. While the pressure decreases, the pressure in the system will at some point be equal to the blood pressure of one of the laboratory animals say at the point $A_1$, and in the corresponding moment $t_2$ the optical sensor 29 associated with that animal will supply an electric signal train to the input of the measuring channel pulsing synchronously with the beats of the heart of the animal.

In the measuring channel (Figure 4) the matching amplifier 33 cuts out the high frequency components of the signal coming from the channel because of its low-pass characteristics with a cut-off frequency of about 400—500 Hz. The low-pass filter 34 consists of two stages with a resultant cut-off rate of 80 dB/decade and a cut-off frequency of about 8 Hz. This high cut-off steepness permits the effective rejection of high frequency noises. The capacitor 32 decouples the DC components from the measuring channel. This choice of filtering range ensures the transmission of signals derived from the pulsation of the laboratory animals being in the range of about 150—480 pulses/minute. The amplifiers 35a, b in the measuring channel amplify the output of the filter signals and the comparator 36 generates pulses from these signals (e.g. by null-comparation) which occur in the form of current pulses in the output of the current generator 37b. These pulses can be seen as vertical spikes on the time-scale used in Figure 5.

When the first impulse is received, the program controller and evaluating unit 40 stores the corresponding pressure in that instant $t_2$. The unit 40 regards this pressure stored in the moment $t_2$ as a valuable measuring datum if the following conditions are met. The program controller and evaluating unit 40 counts the pulses coming in from the measuring channel during six consecutive seconds from the moment $t_2$. If at least four pulses are received in each of the six consecutive seconds, then the pressure value stored in the moment $t_2$ and illustrated in point $A_1$ is considered to be the blood pressure of the animal associated with the concerned measuring channel.

The establishment of the above conditions is important for the elimination of noise signals caused by the trembling and other movements like tail-strokes of the laboratory animals.

In measurement period 1 shown in Figure 5 the blood pressure of all laboratory animals is measured in a similar way and the corresponding data are recorded separately for each channel. The other two measurement periods 2 and 3 shown in Figure 5 illustrate two defective measurements.

When the first measurement period 1 has been completed in the moment $t_3$, another measurement period 2 is started, in which the pressure curve has a shape similar to that of the previous measurement period. At the moment $t_4$ the optical sensor 29 assigned to this channel senses a pulsation caused e.g. by trembling in the point $A_2$ that lasts for a few seconds. The program controller and evaluating unit 40 records the pressure in the moment $t_4$. The normal pulsation of the animal starts at the moment $t_5$ at the point $A_3$, and the signals caused by the trembling and the pulsation, respectively, follow each other continuously. Therefore, the at least four pulses are received during each of the six consecutive periods of 1 second, and the system records the false pressure value prevailing in the point $A_2$ as the actual blood pressure of the animal.

In the third measurement period 3 the pulsation starts at moment $t_6$ associated with point $A_4$ on the pressure curve. Two seconds later due to tailstrokes or the like the pulsation ceases for a short period of time, then it starts again in the moment $t_7$. The pressure in the moment $t_7$ is indicated in the point $A_5$. The program controller and evaluating unit 40 does not take the recorded pressure in the point $A_4$ into account because the conditions have not been met, but it considers the pressure of the point $A_5$ as the actual blood pressure, because the pulsation can be sensed without disturbances after the moment $t_7$. This measurement is also false, since it considers the false value of the point $A_5$ as a result instead of the correct value of the point $A_4$.

It can be seen from the above description that the measurements are carried out separately in the respective channels in consecutive measurement periods common to all channels, in which the actual pressures are the same in all channels. Each measurement period has the same duration $T_1$ and the periods $T_3$ of pressure increasing are also the same for all channels, while only the periods $T_2$ of the pulse occurrence are different between the channels.

Although the application of measuring channels shown in Figure 4 resulted in a considerable increase in noise and false signal rejection, the above described examples illustrate that the application of such measuring channels cannot eliminate in itself the possibility of false measurements.

For the sake of attaining increased reliability, a sequence of measurements is carried out comprising a predetermined number $k$ of measuring periods taken on a number $n$ of laboratory animals, in which $k$ can be varied between about 5 and 20 and it is preferably 16. The data obtained from the respective channels are evaluated by channels as follows: at the end of the $k$ measuring periods $m$ valuable pressure readings will be available in each channel. If $m \leq c \cdot k$ ($c$ being between about 0.7 and 0.8), the sequence is conditionally accepted as good, and the evaluation process can be started. If the above condition is not met, a further sequence is started. New sequences are started a maximum three times, and if the above condition is still not met, an error indication is generated.

If the value obtained for $m$ has satisfied the preset condition, an examination will be carried out on the sequence of the valuable blood pressure values on the basis of mathematical statistics. It will be examined whether the elements of the sequence fall within a given range of deviation of an expectable mean value with a certainty of a predetermined confidence level. For carrying out this examination, the elements falling outside that range will not be considered and it will be determined whether the number of the remaining elements is higher than a further predetermined value (or than $m$). If this condition is met, the mean value of such remaining elements will be considered as the blood pressure of the animal examined by that channel. If this condition is not met, new measurement sequences will be started a maximum of three times.

In each sequence of measurements the evaluation is carried out separately for each channel, and the sequence is repeated, if at least one of the channels request such repetition. For the sake of sparing superfluous evaluations, in case of repeated measurements the evaluation is restricted to the concerned channels only.

By means of the above described technique blood pressure measurements have been carried out with an accuracy better than about 3% in case of rats, and apart from the treatment of hypotensive agents no other treatment was applied.

## Claims

1. Device for measuring the blood pressure of laboratory animals, comprising individual measuring cages $(10_1...10_{12})$ each adapted to accommodate one laboratory animal, combined clamping and optical sensor units $(20_1...20_{12})$ fastenable respectively on the tails of the laboratory animals, measuring channels (22, 30) for processing output signals of the optical sensors, and a program controller and evaluating unit characterised in that said measuring cages $(10_1...10_{12})$ comprise a wall or walls allowing the tails to extend out from the cages, a bottom (11), a heater (12) for heating the bottom (11) and a temperature regulator unit (13) coupled to the heater (12) for adjusting the temperature thereof to a predetermined value, the device further comprising pressure developing and distributing equipment (23), air conduits (21) coupling the individual combined clamping and optical sensor units (20) to said equipment (23) having a common pressure distributor space for all sensor units and a pressure transducer (31) coupled to said common pressure distributor space supplying an electric output signal proportional to the pressure prevailing in said common distributor space, which signal is connected to an input of the programme controller and evaluating unit (40) for supplying a signal, which controls said equipment (23) in order to develop predetermined periodical pressure changes in said common pressure distributor space.

2. The device as claimed in claim 1, in which each measuring channel comprises in series a decoupling capacitor (32), a matching amplifier (33) of low-pass characteristic, a low-pass filter (34) with high cut-off rate and a cut-off frequency under 10 Hz an amplifier (35) connected to the output of the filter (34), a comparator (36) and a coupling unit (37) with DC isolation connected to the output of the comparator.

3. The device as claimed in claim 1, in which the common pressure distributor space of the pressure developing and distributing equipment (23) communicates through an opening of a needle valve with the atmosphere.

4. The device as claimed in any of claims 1 to 3, characterised in that the bottom level of the cages is heatable to a temperature between 33 and 35°C during the measurements, that the program controller and evaluating unit (40) controls the simultaneous increasing and decreasing of the air pressure in said sensor units $(20_1...20_{12})$ repeatedly in a number of consecutive measuring periods $(T_1)$, that the measuring channels each comprise a low-pass filter (34) with a cut-off frequency of not more than 10 Hz, and that the program controller and evaluation unit stores the pressure value $(A_1)$ in each measuring period $(T_1)$ when the first pulse signal of the blood pressure is detected in the interval of decreasing pressure $(T_1—T_3)$ of the measuring period and regards this value as the blood pressure of the laboratory animal if at least a predetermined number of pulse signals have been detected within respective consecutive time sections of given length following the detecting of the first pulse.

## Patentansprüche

1. Vorrichtung zur Messung des Blutdruckes von Labortieren, mit individuellen Meßkäfigen $(10_1...10_{12})$, die jeweils zur Aufnahme eines Labortieres ausgebildet sind, kombinierten Klemm- und Optikfühlereinheiten $(20_1...20_{12})$, die entsprechend an den Schwänzen der Labortiere befestigbar sind, Meßkanälen (22, 30) zur Verarbeitung von Ausgangssignalen der optischen Fühler, und einer Programmsteuer- und Auswerteinheit, dadurch gekennzeichnet, daß die Meßkäfige $(10_1...10_{12})$ eine oder mehrere das Herausragen der Schwänze aus den Käfigen zulassende Wände, einen Boden (11), eine Heizvorrichtung (12) zum Heizen des Bodens (11) und eine Temperatursteuereinheit (13) aufweisen die mit der Heizvorrichtung (12) zur Einstellung von deren Temperatur auf einen vorbestimmten Wert gekoppelt ist, daß die Vorrichtung weiter eine Druckerzeuger- und -verteilereinrichtung (23), Luftleitungen (21), von denen die individuellen kombinierten Klemm- und Optikfühlereinheiten (20) mit der Druckerzeuger- und -verteilereinrichtung (23) gekuppelt werden, welche einen gemeinsamen Druckverteilerraum für alle Fühlereinheiten aufweist, und einen Druckumformer (31) aufweist, der an den gemeinsamen Druckverteilerraum zur Lieferung eines elektrischen Ausgangssignales gekuppelt ist, das proportional zu dem Druck in dem gemeinsamen Verteilerraum ist und an einen Eingang der Programmsteuer und Auswerteinheit (40) zur Lieferung eines Signales abgebbar ist, von welchem die Druckerzeuger- und -verteilereinrichtung (23) zur Herbeiführung von vorbestimmten periodischen Druckänderungen in dem gemeinsamen Druckverteilerraum gesteuert wird.

2. Vorrichtung nach Anspruch 1, bei welcher jeder Meßkanal in Reihe einen Entkopplungskondensator (23), einen Anpaßverstärker (33) mit Tiefpaßeigenschaften, einen Tiefpaßfilter (34) mit einer hohen Cut-Off-Rate und einer Cut-Off-Frequenz unter 10 Hz, einem Verstärker (35), der an den Ausgang des Filters (34) angeschlossen ist, einen Komparator (36) und eine an den Ausgang des Komparators angeschlossene Kopplungseinheit mit Gleichstromtrennung enthält.

3. Vorrichtung nach Anspruch 1, bei welchem der gemeinsame Druckverteilerraum der Druckerzeugungs und -verteilereinrichtung (23) durch eine Öffnung eines Nadelventils hindurch mit der Atmosphäre in Verbindung steht.

4. Vorrichtung nach einem der Ansprüche 1—3, dadurch gekennzeichnet, daß der Boden der Käfige auf eine Temperatur zwischen 33°C und 35°C während der Messungen aufheizbar ist, daß von der Programmsteuer und Auswerteinheit (40) das in einer Anzahl von aufeinander folgenden Meßperioden $(T_1)$ wiederholte gleichzeitige Ansteigen und Abfallen des Luftdruckkes in den Fühlereinheiten $(20_1 \ldots 20_{12})$ gesteuert wird, daß die Meßkanäle jeweils ein Tiefpaßfilter (34) mit einer Cut-Off-Frequenz von nicht mehr als 10 Hz aufweisen und daß von der Programmsteuer- und Auswerteinheit der Druckwert $(A_1)$ in jeder Meßperiode $(T_1)$ gespeichert wird, wenn das erste Pulssignal des Blutdruckes in dem Intervall abfallenden Druckes $(T_1—T_3)$ der Meßperiode abgefühlt wird, und diesen Wert als den Blutdruck des Labortieres ansieht, falls wenigstens eine vorbestimmte Anzahl von Pulssignalen innerhalb entsprechender aufeinander folgender Zeitabschnitte gegebener Länge im Anschluß an das Abtasten des ersten Pulssignales abgetastet wird.

**Revendications**

1. Dispositif de mesure de la pression sanguine d'animaux de laboratoire, comprenant des cages individuelles de mesure $(10_1 \ldots 10_{12})$ dont chacune est adaptée à recevoir un animal de laboratoire, des unités de serrage et de détection optique $(20_1 \ldots 20_{12})$ pouvant être fixées respectivement aux queues des animaux de laboratoire, des canaux de mesure (22, 30) pour traiter les signaux de sortie des détecteurs optiques, et un ensemble de commande de programme et d'évaluation, caractérisé en ce que lesdites cages de mesure $(10_1 \ldots 10_{12})$ comportent une paroi ou des parois permettant aux queues de s'étendre à l'extérieur des cages, un fond (11), un dispositif de chauffage (12) adapté à chauffer le fond (11) et une unité de régulation de température (13) couplée au dispositif de chauffage (12) pour en régler la température à une valeur prédéterminée, le dispositif comprenant, en outre, un équipement de création et de distribution de pression (23), des conduites d'air (21) accouplant les unités de serrage et de détection optique combinées individuelles (20) audit équipement (23) ayant un espace de distribution de pression commun pour toutes les unités détectrices et un transducteur de pression (31) couplé audit espace de distribution de pression commun fournissant un signal de sortie électrique proportionnel à la pression prédominant dans ledit espace de distribution commun, lequel signal est envoyé à l'entrée de l'unité de commande, de programme et d'évaluation (40) pour fournir un signal qui commande ledit équipement (23) en vue de provoquer des modifications de pression périodiques et prédéterminées dans ledit espace de distribution de pression commun.

2. Dispositif selon la revendication 1, dans lequel chacun des canaux de mesure comprend, en série, un condensateur de désaccouplement (32), un amplificateur d'appariement (33) à caractéristique passe-bas, un filtre passe-bas (34) ayant une forte vitesse d'interruption et une fréquence d'interruption inférieure à 10 Hz, un amplificateur (35) réuni à la sortie du filtre (34), un comparateur (36) et une unité d'accouplement (37) avec une isolation au courant continu réunie à la sortie du comparateur.

3. Dispositif selon la revendication dans lequel l'espace de distribution de pression commun de l'équipement de création et de distribution de pression (23) communique par l'ouverture d'une soupape à pointeau avec l'atmosphère.

4. Dispositif selon l'une quelconque des revendications 1 à 3, caractérisé en ce que le niveau du fond des cages est chauffable à une température comprise entre 33 et 35°C pendant les mesures, en ce que l'unité d'évaluation et de commande de programme (40) commande l'augmentation et l'abaissement de la pression de l'air simultanée dans lesdits ensembles détecteurs $(20_1 \ldots 20_{12})$ de manière répétée pendant un nombre de périodes de mesure consécutives $(T_1)$, en ce que les canaux de mesure comprennent chacun un filtre passe-bas (34) avec une fréquence d'interruption n'excédant pas 10 Hz et en ce que l'unité de commande et d'évaluation de programme emmagasine la valeur de pression ($A_1$) pour chaque période de mesure ($T_1$) lorsque le premier signal d'impulsion de la pression sanguine est détecté dans l'intervalle de pression décroissante ($T_1$—$T_3$) de la période de mesure et considère cette valeur comme étant la pression sanguine de l'animal de laboratoire si le nombre de signaux d'impulsion détectés est au moins égal à un nombre prédéterminé pendant les laps de temps consécutifs respectifs de longueur donnée suivant la détection de la première impulsion.

Fig. 1

Fig. 2

Fig. 3

Fig. 5

Fig. 4